# EUROPEAN PATENT SPECIFICATION

(11) **EP 1 477 497 B1**
(45) Date of publication and mention of the grant of the patent: **26.11.2014**
(21) Application number: 04076439.1
(22) Date of filing: 28.12.1989
(51) Int. Cl.: C07K 16/00, C07K 16/46, C12N 5/10

(54) **Method for the production of humanized immunoglobulins**
Verfahren zur Herstellung von humanisierten Immunglobulinen
Procédé de production d' anticorps humanisés

(30) Priority: 28.12.1988 US 290975; 13.02.1989 US 310252
(43) Date of publication of application: 17.11.2004
(62) Divisional of application: 98204240.0
(73) Proprietor: PDL BioPharma, Inc., Incline Village, NV 89451 (US)
(72) Inventor: Queen, Cary L., Cupertino, CA 95014 (US); Selick, Harold E., Belmont, CA 94002 (US)
(74) Representative: Wilkinson, Marc George

(56) References cited:
- EP-A- 0 328 404
- EP-A2- 0 278 160
- WO-A1-89/09622
- RIECHMANN L. ET AL: "Reshaping human antibodies for therapy" NATURE, vol. 332, 24 March 1988 (1988-03-24), pages 323-327, XP002949268 LONDON GB
- QUEEN C. ET AL: "A humanised antibody that binds to the interleukin 2 receptor" PROCEEDINGS OF THE NATIONAL ACADEMY OF SCIENCES OF USA, vol. 86, December 1999 (1999-12), pages 10029-10033, XP002947922 WASHINGTON US
- MCCABE R P ET AL: "PRECLINICAL STUDIES ON THE PHARMACOKINETIC PROPERTIES OF HUMAN MONOCLONAL ANTIBODIES TO COLORECTAL CANCER AND THEIR USE FOR DETECTION OF TUMORS", CANCER RESEARCH, AMERICAN ASSOCIATION FOR CANCER RESEARCH, US, vol. 48, no. 15, 1 January 1988 (1988-01-01), pages 4348-4353, XP002631000, ISSN: 0008-5472
- KOZAK R W ET AL: "Bismuth-212-labeled anti-Tac monoclonal antibody: alpha-particle-emitting radionuclides as modalities for radioimmunotherapy.", PROCEEDINGS OF THE NATIONAL ACADEMY OF SCIENCES OF THE UNITED STATES OF AMERICA JAN 1986 LNKD- PUBMED:3079913, vol. 83, no. 2, January 1986 (1986-01), pages 474-478, ISSN: 0027-8424

## Description

### Field of the Invention

The present invention relates generally to the combination of recombinant DNA and monoclonal antibody technologies for developing novel therapeutic agents and, more particularly, to the production of non-immunogenic antibodies and their uses.

### Background of the Invention

In mammals, the immune response is mediated by two types of cells that interact specifically with foreign material, i.e., antigens. One of these cell types, B-cells, are responsible for the production of antibodies. The second cell class, T-cells, include a wide variety of cellular subsets controlling the in vivo function of both B-cells and a wide variety of other hematopoietic cells, including T-cells.

One way in which T-cells exert this control is through the production of a lymphokine known as interleukin-2 (IL-2), originally named T-cell growth factor. IL-2's prime function appears to be the stimulation and maintenance of T-cells. Indeed, some immunologists believe that IL-2 may be at the center of the entire immune response (see, Farrar, J., et al., Immunol. Rev. 63:129-166 (1982), which is incorporated herein by reference).

To exert its biological effects, IL-2 interacts with a specific high-affinity membrane receptor (Greene, W., et al., Progress in Hematology XIV, E. Brown, Ed., Grune and Statton, New York (1986), at pgs. 283 ff). The human IL-2 receptor is a complex multichain glycoprotein, with one chain, known as the Tac peptide, being about 55kD in size (see, Leonard, W., et al., J. Biol. Chem. 260:1872 (1985), which is incorporated herein by reference). A gene encoding this protein has been isolated, and predicts a 272 amino acid peptide, including a 21 amino acid signal peptide (see, Leonard, W., et al., Nature 311: 626 (1984)). The 219 NH₂-terminal amino acids of the p55 Tac protein apparently comprise an extracellular domain (see, Leonard, W., et al., Science, 230:633-639 (1985), which is incorporated herein by reference).

Much of the elucidation of the human IL-2 receptor's structure and function is due to the development of specifically reactive monoclonal antibodies. In particular, one mouse monoclonal antibody, known as anti-Tac (Uchiyama, et al., J. Immunol. 126:1393 (1981)) has shown that IL-2 receptors can be detected on T-cells, but also on cells of the monocyte-macrophage family, Kupffer cells of the liver, Langerhans' cells of the skin and, of course, activated T-cells. Importantly, resting T-cells, B-cells or circulating machrophages typically do not display the IL-2 receptor (Herrmann, et al., J. Exp. Med. 162:1111 (1985)).

The anti-Tac monoclonal antibody has also been used to define lymphocyte functions that require IL-2 interaction, and has been shown to inhibit various T-cell functions, including the generation of cytotoxic and suppressor T lymphocytes in cell culture. Also, based on studies with anti-Tac and other antibodies, a variety of disorders are now associated with improper IL-2 receptor expression by T-cells, in particular adult T-cell leukemia.

More recently, the IL-2 receptor has been shown to be an ideal target for novel therapeutic approaches to T-cell mediated diseases. It has been proposed that IL-2 receptor specific antibodies, such as the anti-Tac monoclonal antibody, can be used either alone or as an immunoconjugate (e.g., with Ricin A, isotopes and the like) to effectively remove cells bearing the IL-2 receptor. These agents can, for example, theoretically eliminate IL-2 receptor-expressing leukemic cells, certain B-cells, or activated T-cells involved in a disease state, yet allow the retention of mature normal T-cells and their precursors to ensure the capability of mounting a normal T-cell immune response as needed. In general, most other T-cell specific agents can destroy essentially all peripheral T-cells, which limits the agents' therapeutic efficacy. Overall, the use of appropriate monoclonal antibodies specific for the IL-2 receptor may have therapeutic utility in autoimmune diseases, organ transplantation and- any unwanted response by activated T-cells. Indeed, clinical trials have been initiated using, e.g., anti-Tac antibodies (see, generally, Waldman, T., et al., Cancer Res. 45:625 (1985) and Waldman, T., Science 232:727-732 (1986), both of which are incorporated herein by reference).

Unfortunately, the use of the anti-Tac and other non-human monoclonal antibodies have certain drawbacks, particularly in repeated therapeutic regimens as explained below. Mouse monoclonal antibodies, for example, do not fix human complement well, and lack other important immunoglobulin functional characteristics when used in humans.

Perhaps more importantly, anti-Tac and other non-human monoclonal antibodies contain substantial stretches of amino acid sequences that will be immunogenic when injected into a human patient. Numerous studies have shown that, after injection of a foreign antibody, the immune response elicited by a patient against an antibody can be quite strong, essentially eliminating the antibody's therapeutic utility after an initial treatment. Moreover, as increasing numbers of different mouse or other antigenic (to humans) monoclonal antibodies can be expected to be developed to treat various diseases, after the first and second treatments with any different non-human antibodies, subsequent treatments even for unrelated therapies can be ineffective or even dangerous in themselves.

While the production of so-called "chimeric antibodies" (e.g., mouse variable regions joined to human constant regions) (see, for example, WO89/09622) has proven somewhat successful, a significant immunogenicity problem remains. In general, the production of human immunoglobulins reactive with the human IL-2 receptor, as with many human antigens, has been extremely difficult using typical human monoclonal antibody production techniques. Similarly, past attempts utilizing recombinant DNA technology to produce so-called "humanized" antibodies (see e.g., EPO Publication) No. 0239400), provides uncertain results, in part due to unpredictable binding affinities.

Thus, there is a need for improved forms of human-like immunoglobulins, such as those specific for the human IL-2 receptor, that are substantially non-immunogenic in humans, yet easily and economically produced in a manner suitable for therapeutic formulation and other uses. The present invention fulfills these and other needs. The hypervariable regions (also called Complementarity Determining Regions, abbreviated to "CDRs") of immunoglobulins were originally defined by Kabat et al., ("Sequences of Proteins of Immunological Interest" Kabat, E., et al., U.S. Department of Health and Human Services, (1983)) based on extent of sequence variability, to consist of residues 24-34 (L1), 50-56 (L2) and 89-97 (L3) in the light chain variable domain (V_{L}) and 31-35 (H1), 50-65 (H2) and 95-102 (H3) in the heavy chain variable domain (V_{H}) , using Kabat's standard numbering system for antibody amino acids. The CDRs are believed to contact the target antigen of an antibody and to be primarily responsible for binding. More recently Chothia et al (Chothia and Lesk, J. Mol. Biol., 196:901-917 (1987)) have given an alternate definition of the hypervariable regions as consisting of residues 26-32 (L1), 50-52 (L2), 91-96 (L3) in V_{L} and residues 26-32 (H1), 53-55 (H2), 96-101 (H3) in V_{H}. The Chothia definition is based on the residues that constitute the loops in the 3-dimensional structures of antibodies. It is particularly important to note that for each of the six CDRs the Chothia hypervariable region is actually a subset of (i.e., smaller than) the ,Kabat CDR, with the single exception of H1 (the first heavy chain CDR), where the Chothia hypervariable region contains amino acids 26-30 that are not in the Kabat CDR.

Reichmann et al ("Reshaping human antibodies for therapy", Nature, Vol 332, pp 323-326, (March 1988)) describe work in which precisely the Kabat CDRs were transferred to a pre-determined human framework (NEW again for the heavy chain and REI for the light chain). However, they found that antibody containing the humanized heavy chain lost most of its binding affinity and ability to lyse target cells. They therefore made a new humanized antibody containing the Kabat CDRs from the mouse antibody and two amino acid changes in Chothia hypervariable region H1, but no other mouse amino acids.

### Summary of the Invention

The invention provides a process for producing a humanized immunoglobulin suitable for an immunotoxin in which immunotoxin said immunoglobulin is chemically bonded to a cytotoxic agent, wherein said humanized immunoglobulin binds to an antigen and has a human acceptor framework region and Kabat complementarity determining regions (CDRs) from a donor immunoglobulin, further wherein the production of said humanized immunoglobulin comprises substituting at least one non-CDR amino acid from the donor immunoglobulin light chain framework for the corresponding amino acid in the acceptor framework, at a position where:
(a) the amino acid in the acceptor framework is rare for said position and the corresponding amino acid in the donor framework is common for said position in human immunoglobulin sequences; or
(b) the amino acid is immediately adjacent to one of the CDRs; or
(c) the amino acid is predicted to have a side chain atom capable of interacting with the CDRs of the humanized immunoglobulin.

Humanized immunoglobulins produced in accordance with the principles of the invention can be used as pharmaceuticals, and in the manufacture of medicaments.

The present application describes novel compositions useful, for example, in the treatment of T-cell mediated human disorders, the compositions containing human-like immunoglobulins specifically capable of blocking the binding of human IL-2 to its receptor and/or capable of binding to the p55 Tac protein on human IL-2 receptors. The immunoglobulins can have two pairs of light chain/heavy chain complexes, typically at least one pair having chains comprising mouse complementarity determining regions functionally joined to human framework region segments. For example, mouse complementarity determining regions, with our without additional naturally-associated mouse amino acid residues, can be used to produce human-like antibodies capable of binding to the human IL-2 receptor at affinity levels stronger than about 10⁸ M⁻¹.

Such compositions for use as pharmaceuticals and the use of such compositions in the manufacture of medicaments are also disclosed herein.

The immunoglobulins, including binding fragments and other derivatives thereof, may be produced readily by a variety of recombinant DNA techniques, with ultimate expression in transfected cells, preferably immortalized eukaryotic cells, such as myeloma or hybridoma cells. Polynucleotides comprising a first sequence coding for human-like immunoglobulin framework regions and a second sequence set coding for the desired immunoglobulin complementarity determining regions can be produced synthetically or by combining appropriate cDNA and genomic DNA segments.

The human-like immunoglobulins may be complexed with a cytotoxic agents, such as a radionuclide, a ribosomal inhibiting protein or a cytotoxic agent active at cell surfaces. All of these compounds will be particularly useful in treating T-cell mediated disorders. The human-like immunoglobulins or their complexes can be prepared in a pharmaceutically accepted dosage form, which will vary depending on the mode of administration. The present invention involves novel methods for designing human-like immunoglobulin chains having one or more complementarity determining regions (CDR's) from a donor immunoglobulin and a framework region from a human immunoglobulin, the preferred methods may involve first comparing the framework or variable region amino acid sequence of the donor immunoglobulin to corresponding sequences in a collection of human immunoglobulin chains, and selecting as the human immunoglobulin one of the more homologous sequences from the collection. The human immunoglobulin, or acceptor immunoglobulin, sequence is typically selected from a collection of at least 10 to 20 immunoglobulin chain sequences, and usually will have the highest homology to the donor immunoglobulin sequence of any sequence in the collection. The human immunoglobulin framework sequence will typically have about 65 to 70% homology or more to the donor immunoglobulin framework sequences. The donor immunoglobulin may be either a heavy chain or light chain (or both), and the human collection will contain the same kind of chain. A humanized light and heavy chain can be used to form a complete humanized immunoglobulin or antibody, having two light/heavy chain pairs, with or without partial or full-length human constant regions and other proteins.

Whether or not in conjunction with the above comparison step, additional amino acids in an acceptor immunoglobulin chain may be replaced with amino acids from the CDR-donor immunoglobulin chain. More specifically, further optional substitutions of a human framework amino acid of the acceptor immunoglobulin with a corresponding amino acid from a donor immunoglobulin will be made at positions in the immunoglobulins where:
(a) the amino acid in the human framework region of an acceptor immunoglobulin is rare for that position and the corresponding amino acid in the donor immunoglobulin is common for that position in human immunoglobulin sequences; or
(b) the amino acid is immediately adjacent to one of the CDR's; or
(c) the amino acid is predicted to have a side chain atom capable of interacting with the antigen or with the CDR's of the humanized immunoglobulin, optionally within about 3Å of the CDR's in a three-dimensional immunoglobulin model.

The humanized immunoglobulin chain will typically comprise at least about 3 amino acids from the donor immunoglobulin in addition to the CDR's, usually at least one of which is immediately adjacent to a CDR in the donor immunoglobulin. The heavy and light chains may each be designed by using any one or all three of the position criteria.

When combined into an intact antibody, the humanized light and heavy chains obtained by the present invention will be substantially non-immunogenic in humans and retain substantially the same affinity as the donor immunoglobulin to the antigen (such as a protein or other compound containing an epitope). These affinity levels can vary from about 10⁸ M⁻¹ or higher, and may be within about 4 fold of the donor immunoglobulin's original affinity to the antigen.

### BRIEF DESCRIPTION OF THE FIGURES

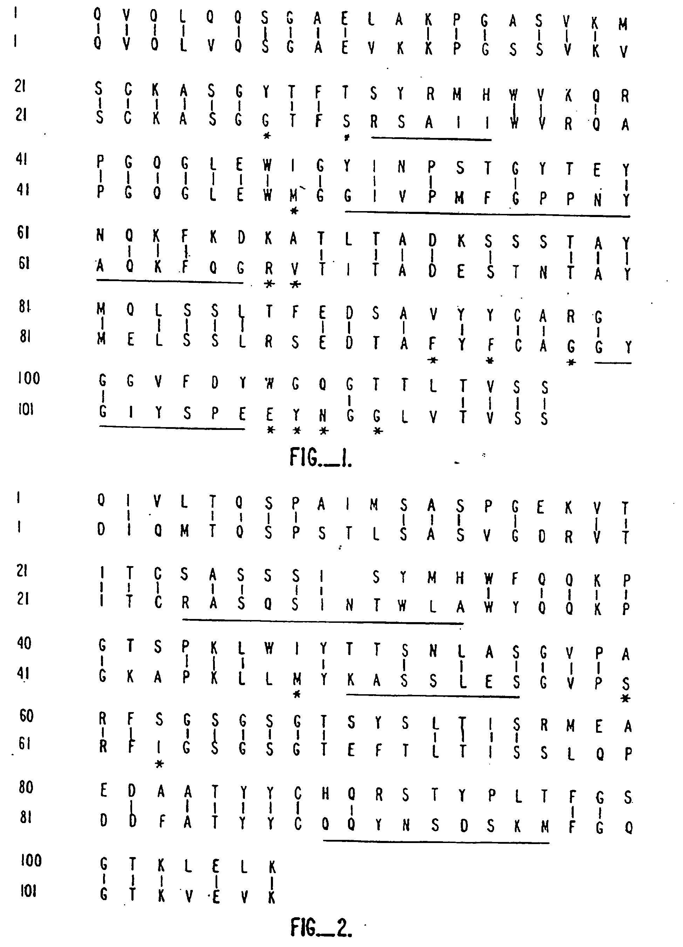

Figure 1. comparison of sequences of anti-Tac heavy chain (upper lines) and Eu heavy chain (lower lines). The 1-letter code for amino acids is used. The first amino acid on each line is numbered at the left. Identical amino acids in the two sequences are connected by lines. The 3 CDRs are underlined. Other amino acid positions for which the anti-Tac amino acid rather than the Eu amino acid was used in the humanized anti-Tac heavy chain are denoted by an *.

Figure 2. Comparison of sequences of anti-Tac light chain (upper lines) and Eu light chain (lower lines). The single-letter code for amino acids is used. The first amino acid on each line is numbered at the left. Identical amino acids in the two sequences are connected by lines. The 3 CDRs are underlined. Other amino acid positions for which the anti-Tac amino acid rather than the Eu amino acid was used in the humanized anti-Tac heavy chain are denoted by an *.

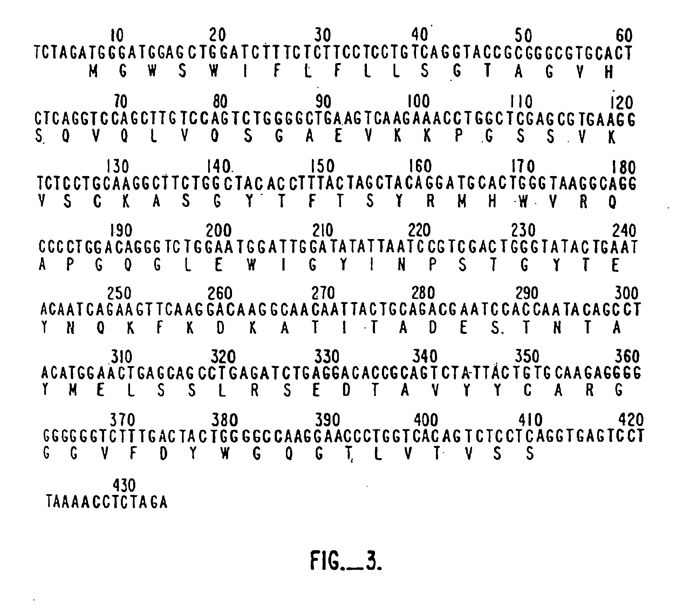

Figure 3. Nucleotide sequence of the gene for the humanized anti-Tac heavy chain variable region gene. The translated amino acid sequence for the part'of the gene encoding protein is shown underneath the nucleotide sequence. The nucleotides TCTAGA at the beginning and end of the gene are Xba I sites. The mature heavy chain sequence begins with amino acid #20 Q.

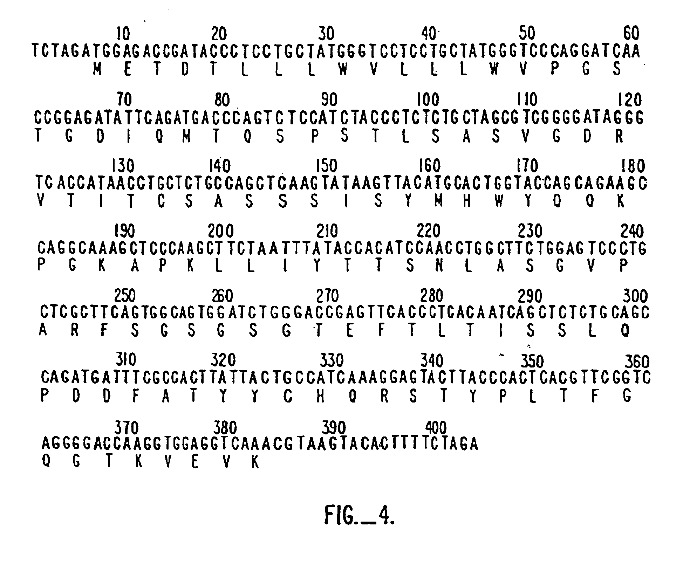

Figure 4. Nucleotide sequence of the gene for the humanized anti-Tac light chain variable region gene. The translated amino acid sequence for the part of the gene encoding protein is shown underneath the nucleotide sequence. The nucleotides TCTAGA at the beginning and end of the gene are Xba I sites. The mature light chain sequence begins with amino acid #21 D.

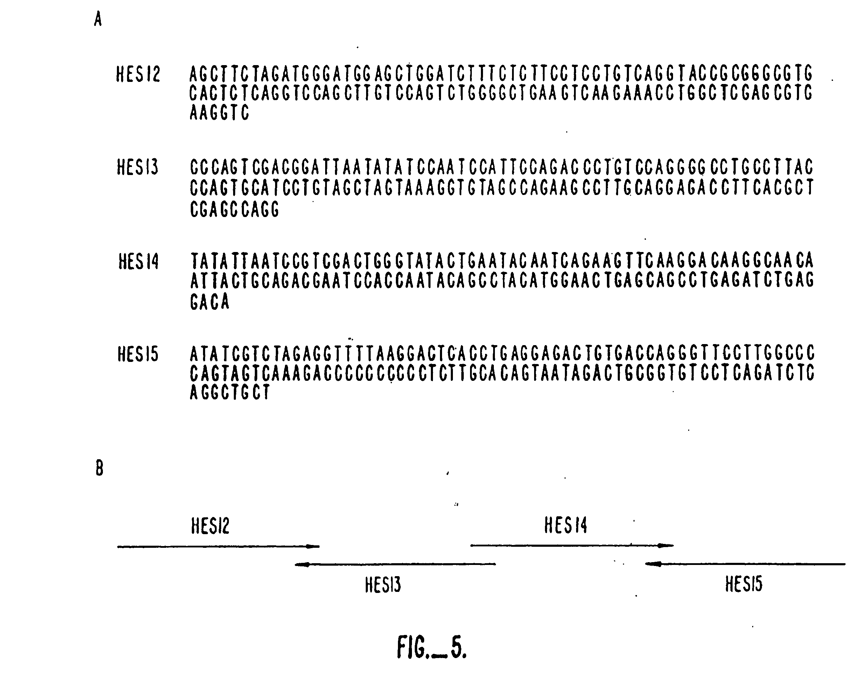

Figure 5. A. Sequences of the four oligonucleotides used to synthesize the humanized anti-Tac heavy chain gene, printed 5' to 3'. B. Relative positions of the oligonucleotides. The arrows point in the 3' direction for each oligonucleotide.

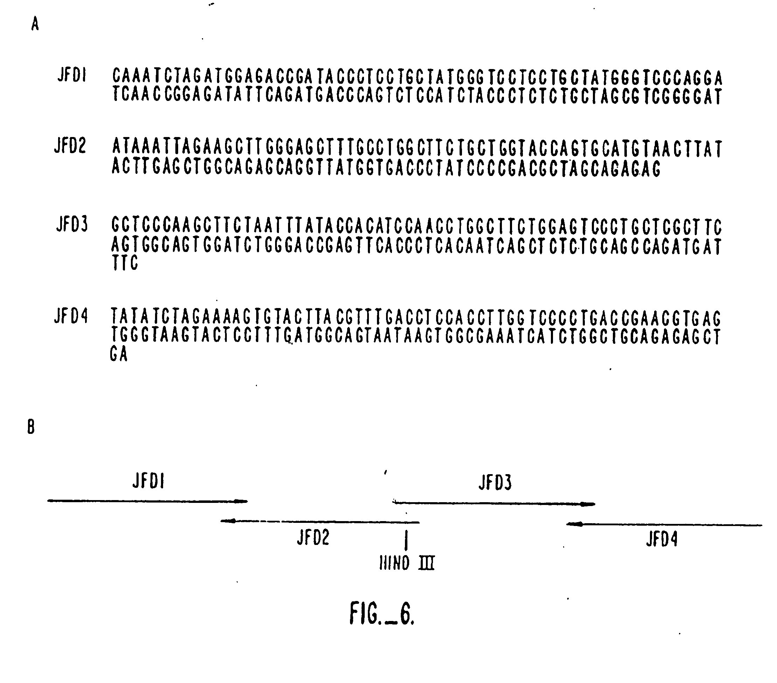

Figure 6. (A) Sequences of the four oligonucleotides used to synthesize the humanized anti-Tac light chain gene, printed 5' to 3'. (B) Relative positions of the oligonucleotides. The arrows point in the 3' direction for each oligonucleotide. The position of a Hind III site in the overlap of JFD2 and JFD3 is shown.

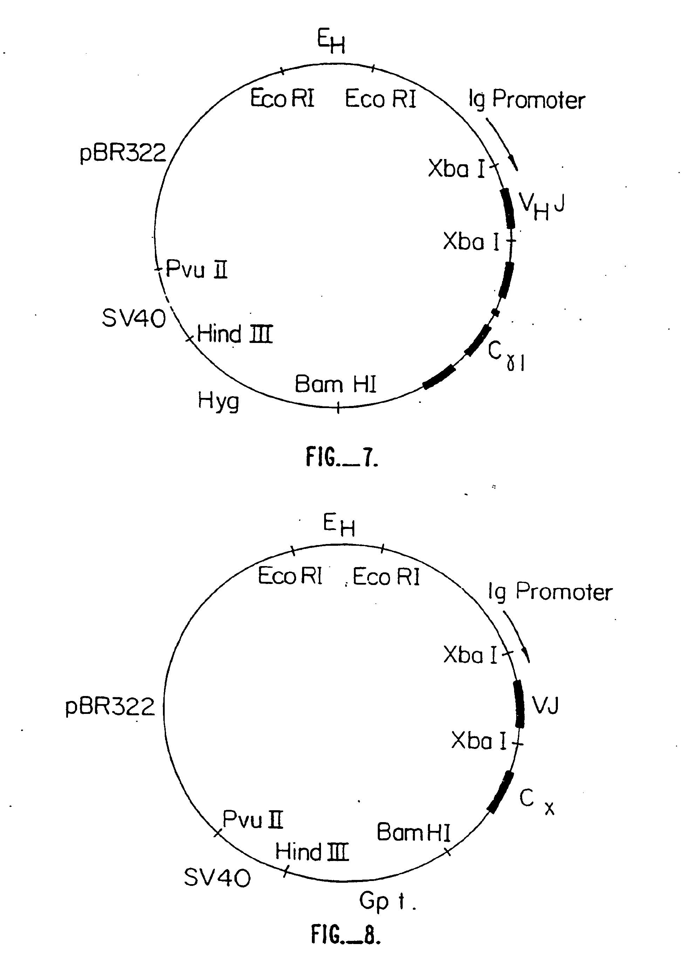

Figure 7. Schematic diagram of the plasmid pHuGTAC1 used to express the humanized anti-Tac heavy chain. Relevant restriction sites are shown, and coding regions of the heavy chain are displayed as boxes. The direction of transcription from the immunoglobulin (Ig) promoter is shown by an arrow. E_{H} = heavy chain enhancer, Hyg = hygromycin resistance gene.

Figure 8. Schematic diagram of the plasmid pHuLTAC used to express the humanized anti-Tac light chain. Relevant restriction sites are shown, and coding regions of the light chain are displayed as boxes. The direction of transcription from the Ig promoter is shown by an arrow.

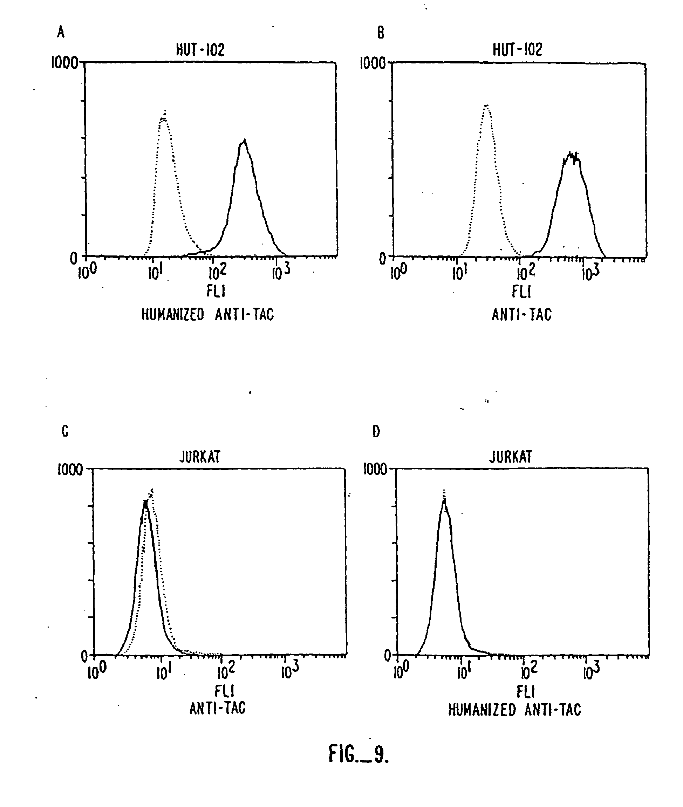

Figure 9. Fluorocytometry of HUT-102 and Jurkat cells stained with anti-Tac antibody or humanized anti-Tac antibody followed respectively by fluorescein-conjugated goat anti-mouse Ig antibody or goat anti-human Ig antibody, as labeled. In each panel, the dotted curve shows the results when the first antibody was omitted, and the solid curve the results when first and second (conjugated) antibodies were included as described.

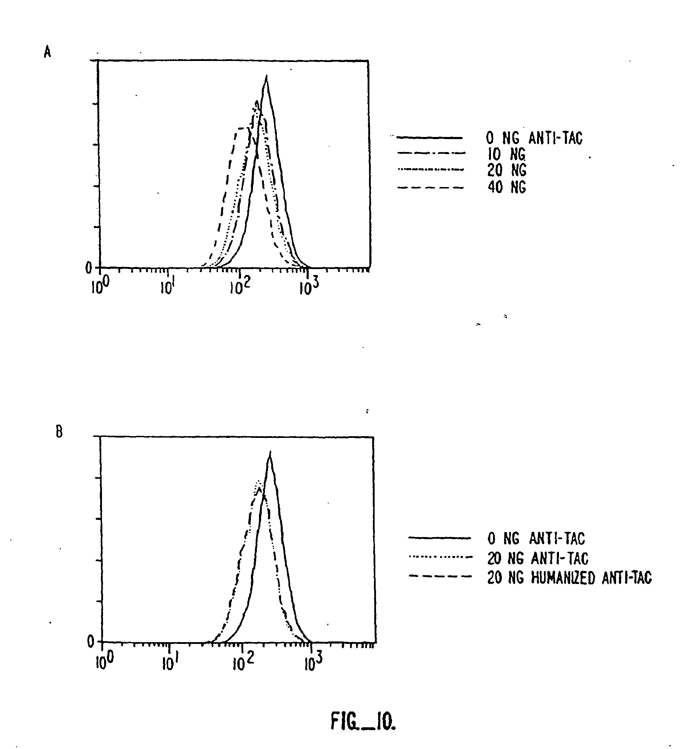

Figure 10. (A) Fluorocytometry of HUT-102 cells stained with 0-40 ng of anti-Tac as indicated, then with biotinylated anti-Tac, and then with phycoerythrin-conjugated avidin. (B) Fluorocytometry of HUT-102 cells stained with the indicated antibody, then with biotinylated anti-Tac, and then with phycoerythrin-conjugated avidin.

### DETAILED DESCRIPTION OF THE INVENTION

In accordance with one embodiment of the present invention, human-like immunoglobulins specifically reactive with desired epitopes, such as those on the IL-2 receptor on human T-cells, are provided. These immunoglobulins, which have binding affinities of at least about 10⁸ M⁻¹, and preferably 10⁹ M⁻¹ to 10¹⁰ M⁻¹ or stronger, are capable of, e.g., blocking the binding of IL-2 to human IL-2 receptors. The human-like immunoglobulins will have a human-like framework and can have complementarity determining regions (CDR's) from an immunoglobulin, typically a mouse immunoglobulin, specifically reactive with an epitope on p55 Tac protein. The immunoglobulins of the present invention, which can be produced economically in large quantities, find use, for example, in the treatment of T-cell mediated disorders in human patients by a variety of techniques.

The basic antibody structural unit is known to comprise a tetramer. Each tetramer is composed of two identical pairs of polypeptide chains, each pair having one "light" (about 25kD) and one "heavy" chain (about 50-70kD). The NH₂-terminus of each chain begins a variable region of about 100 to 110 or more amino acids primarily responsible for antigen recognition. The C00H terminus of each chain defines a constant region primarily responsible for effector function.

Light chains are classified as either kappa or lambda. Heavy chains are classified (and subclassified) as gamma, mu, alpha, delta, or epsilon, and define the antibody's isotype as IgG, IgM, IgA, IgD and IgE, respectively. Within light and heavy chains, the variable and constant regions are joined by a "J" region of about 12 or more amino acids, with the heavy chain also including a "D" region of about 12 more amino acids. (See, generally, Fundamental Immunology, Paul, W., Ed., Chapter 7, pgs. 131-166, Raven Press, N.Y. (1984)).

The variable regions of each light/heavy chain pair form the antibody binding site. The chains all exhibit the same general structure of relatively conserved framework regions joined by three hypervariable regions, also called CDR's (see, "Sequences of Proteins of Immunological Interest," Kabat, E., et al., U.S. Department of Health and Human Services, (1983); and Chothia and Lesk, J. Mol. Biol., 196:901-917 (1987)). The CDR's from the two chains of each pair are aligned by the framework regions, enabling binding to a specific epitope.

As used herein, the term "immunoglobulin" refers to a protein consisting of one or more polypeptides substantially encoded by immunoglobulin genes. The recognized immunoglobulin genes include the kappa, lambda, alpha, gamma, delta, epsilon and mu constant region genes, as well as the myriad immunoglobulin variable region genes. The immunoglobulins may exist in a variety of forms besides antibodies; including, for example, Fv, Fab, and F(ab)₂, as well as in single chains (e.g., Huston, et al., Proc. Nat. Acad. Sci. U.S.A., 85:5879-5883 (1988) and Bird, et al., Science, 242:423-426 (1988), Hood, et al., "Immunology", Benjamin, N.Y., 2nd ed. (1984), and Hunkapiller and Hood, Nature, 323:15-16 (1986)).

Chimeric antibodies are antibodies whose light and heavy chain genes have been constructed, typically by genetic engineering, from immunoglobulin gene segments belonging to different species. For example, the variable (V) segments of the genes from a mouse monoclonal antibody may be joined to human constant (C) segments, such as γ₁ and γ₃. A typical therapeutic chimeric antibody is thus a hybrid protein consisting of the V or antigen-binding domain from a mouse antibody and the C or effector domain from a human antibody (e.g., A.T.C.C. Accession No. CRL 9688 secretes an anti-Tac chimeric antibody), although other mammalian species may be used.

As used herein, the term "framework region" refers to those portions of immunoglobulin light and heavy chain variable regions that are relatively conserved (i.e., other than the CDR's) among different immunoglobulins in a single species, as defined by kabat , et al., op. cit. As used herein, a "human-like framework region" is a framework region that in each existing chain comprises at least about 70 or more amino acid residues, typically 75 to 85 or more residues, identical to those in a human immunoglobulin.

As used herein, the term "human-like immunoglobulin" refers to an immunoglobulin comprising a human-like framework and in which any constant region present is substantially homologous to a human immunoglobulin constant region; i.e., at least about 85-90%, preferably about 95% identical. Hence, all parts of a human-like immunoglobulin, except possibly the CDR's, are substantially homologous to corresponding parts of one or more native human immunoglobulin sequences. For example, a human-like immunoglobulin would not encompass a chimeric mouse variable region/human constant region antibody.

In accordance with another general aspect of the present invention, also included are criteria by which a limited number of amino acids in the framework of a human-like or humanized immunoglobulin chain are chosen to be the same as the amino acids at those positions in the donor Ig rather than in the acceptor Ig, in order to increase the affinity of an antibody comprising the humanized immunoglobulin chain.

This aspect of the present invention is based in part on the model that two contributing causes of the loss of affinity in prior means of producing humanized antibodies (using as examples mouse antibodies as the source of CDR's) are:
(1) When the mouse CDR's are combined with the human framework, the amino acids in the framework close to the CDR's become human instead of mouse. Without intending to be bound by theory, we believe that these changed amino acids may slightly distort the CDR's, because they create different electrostatic or hydrophobic forces than in the donor mouse antibody, and the distorted CDR's may not make as effective contacts with the antigen as the CDR's did in the donor antibody;
(2) Also, amino acids in the original mouse antibody that are close to, but not part of, the CDR's (i.e., still part of the framework), may make contacts with the antigen that contribute to affinity. These amino acids are lost when the antibody is humanized, because all framework amino acids are made human.

To avoid these problems, and to produce humanized antibodies that have a very strong affinity for a desired antigen, the present invention uses the following four criteria for designing humanized immunoglobulins. These criteria may be used singly, or when necessary in combination, to achieve the desired affinity or other characteristics.

Criterion I: As acceptor, use a framework from a particular human immunoglobulin that is unusually homologous to the donor immunoglobulin to be humanized, or use a consensus framework from many human antibodies. For example, comparison of the sequence of a mouse heavy (or light) chain variable region against human heavy (or light) variable regions in a data bank (for example, the National Biomedical Research Foundation Protein Identification Resource) shows that the extent of homology to different human regions varies greatly, typically from about 40% to about 60-70%. By choosing as the acceptor immunoglobulin one of the human heavy (respectively light) chain variable regions that is most homologous to the heavy (respectively light) chain variable region of the donor immunoglobulin, fewer amino acids will be changed in going from the donor immunoglobulin to the humanized immunoglobulin. Hence, and again without intending to be bound by theory, it is believed that there is a smaller chance of changing an amino acid near the CDR's that distorts their conformation. Moreover, the precise overall shape of a humanized antibody comprising the humanized immunoglobulin chain may more closely resemble the shape of the donor antibody, also reducing the chance of distorting the CDR's.

Typically, one of the 3-5 most homologous heavy chain variable region sequences in a representative collection of at least about 10 to 20 distinct human heavy chains will be chosen as acceptor to provide the heavy chain framework, and similarly for the light chain. Preferably, one of the 1-3 most homologous variable regions will be used. The selected acceptor immunoglobulin chain will most preferably have at least about 65% homology in the framework region to the donor immunoglobulin.

Regardless of how the acceptor immunoglobulin is chosen, higher affinity may be achieved by selecting a small number of amino acids in the framework of the humanized immunoglobulin chain to be the same as the amino acids at those positions in the donor rather than in the acceptor. The following criteria define what amino acids may be so selected. Preferably, at most or all amino acid positions satisfying one of these criteria, the donor amino acid will in fact be selected.

Criterion II: If an amino acid in the framework of the human acceptor immunoglobulin is unusual (i.e., "rare", which as used herein indicates an amino acid occurring at that position in no more than about 10% of human heavy (respectively light) chain V region sequences in a representative data bank), and if the donor amino acid at that position is typical for human sequences (i.e., "common", which as used herein indicates an amino acid occurring in at least about 25% of sequences in a representative data bank), then the donor amino acid rather than the acceptor may be selected. This criterion helps ensure that an atypical amino acid in the human framework does not disrupt the antibody structure. Moreover, by replacing an unusual amino acid with an amino acid from the donor antibody that happens to be typical for human antibodies, the humanized antibody may be made less immunogenic.

Criterion III: In the positions immediately adjacent to the 3 CDR's in the humanized immunoglobulin chain, the donor amino acid rather than acceptor amino acid may be selected. These amino acids are particularly likely to interact with the amino acids in the CDR's and, if chosen from the acceptor, distort the donor CDR's and reduce affinity. Moreover, the adjacent amino acids may interact directly with the antigen (Amit et al., Science, 233, 747-753 (1986)). and selecting these amino acids from the donor may be desirable to keep all the antigen contacts that provide affinity in the original antibody.

Criterion IV: A 3-dimensional model, typically of the original donor antibody, shows that certain amino acids outside of the CDR's are close to the CDR's and have a good probability of interacting with amino acids in the CDR's by hydrogen bonding, Van der Waals forces, hydrophobic interactions, etc. At those amino acid positions, the donor amino acid rather than the acceptor immunoglobulin amino acid may be selected. Amino acids according to this criterion will generally have a side chain atom within about 3 angstrom units of some site in the CDR's and must contain atoms that could interact with the CDR atoms according to established chemical forces, such as those listed above. Computer programs to create models of proteins such as antibodies are generally available and well known to those skilled in the art (see, Loew et al., Int. J. Quant. Chem., Quant. Biol. Symp., 15:55-66 (1988); Bruccoleri et al., Nature, 335, 564-568 (1988); Chothia et al., Science, 233:755-758 (1986)). These do not form part of the invention. Indeed, because all antibodies have similar structures, the known antibody structures, which are available from the Brookhaven Protein Data Bank, can be used if necessary as rough models of other antibodies. Commercially available computer programs can be used to display these models on a computer monitor, to calculate the distance between atoms, and to estimate the likelihood of different amino acids interacting (see, Ferrin et al., J. Mol. Graphics, 6 : 13-27 (1988)).

Humanized or human-like antibodies generally have at least three potential advantages over mouse or in some cases chimeric antibodies for use in human therapy:
1) Because the effector portion is human, it may interact better with the other parts of the human immune system (e.g., destroy the target cells more efficiently by complement- dependent cytotoxicity (CDC) or antibody-dependent cellular cytotoxicity (ADCC)).
2) The human immune system should not recognize the framework or constant region of the humanized antibody as foreign, and therefore the antibody response against such an injected antibody should be less than against a totally foreign mouse antibody or a partially foreign chimeric antibody.
3) Injected mouse antibodies have been reported to have a half-life in the human circulation much shorter than the half-life of normal antibodies (D. Shaw et al., J. Immunol., 138:4534-4538 (1987)). Injected humanized antibodies will presumably have a half-life more similar to naturally occurring human antibodies, allowing smaller and less frequent doses to be given.

The present invention is specifically directed to methods for making improved humanized immmunoglobulins (e.g., capable of binding the human IL-2 receptor) with respect to those described in EPA publication no. 0239400. That application, the disclosure of which is excluded from coverage herein, describes, for certain immunoglobulins, substituting CDR's regions in the light or heavy chain variable domains of an acceptor antibody with analogous parts of CDR's (typically solvent accessible) from an antibody of different specificity. Also, that application discusses, for certain immunoglobulins, the possibility of only transferring residues that are (solvent) accessible from the antigen binding site, which residues apparently may include certain framework regions (specifically, residues known to be involved in antigen binding as described in Amit et al., Science 233: 747-753 (1986) or perhaps residues essential for inter-chain interactions - but for the selection of which insufficient guidance is provided-in that application). Thus, for example, a preferred embodiment of the present invention entails substituting entire CDR's and framework amino acids immediately adjacent one (or preferably each) of the CDR's. In general, any framework residue that also makes contact with the CDR's to, e.g., maintain their conformation (and usually their antigen binding specificity) are specifically included within preferred embodiments of the present invention as described in detail, supra.

In one aspect, the present invention involves recombinant DNA segments encoding the heavy and/or light chain CDR's (typically with other amino acid residues as described above) from an immunoglobulin capable of binding to a desired epitope, such as on the human IL-2 receptor (e.g., the anti-Tac monoclonal antibody). The DNA segments encoding these regions will typically be joined to DNA segments encoding appropriate human-like framework regions. For example, the preferred DNA sequences, which on expression code for the polypeptide chains comprising the anti-Tac heavy and light chain hypervariable regions (with human-like framework regions), are shown in Figures 3 and 4, respectively. Due to codon degeneracy and non-critical amino-acid substitutions, other DNA sequences can be readily substituted for those sequences, as detailed below.

The DNA segments will typically further include an expression control DNA sequence operably linked to the human-like antibody coding sequences, including naturally-associated or heterologous promoter regions. Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the light chains, heavy chains, light/heavy chain dimers or intact antibodies, binding fragments or other immunoglobulin forms may follow.

Human constant region DNA sequences can be isolated in accordance with well known procedures from a variety of human cells, but preferably immortalized B-cells (see, Kabat op. cit. and WP87/02671). For example, the human kappa immunoglobulin constant and J region genes and sequences are described in Heiter et al., Cell 22:197-207 (1980) and the nucleotide sequence of a human immunoglobulin C_{γ1} gene is described in Ellison et al., Nucl. Acid. Res. 10:4071 (1982)). The CDR's for producing the immunoglobulins of the present invention will be similarly derived from monoclonal antibodies capable of binding to the desired antigen (e.g., the human IL-2 receptor) and produced in any convenient mammalian source, including, mice, rats, rabbits, or other veterbrate capable of producing antibodies by well known methods. Suitable source cells for the DNA sequences and host cells for immunoglobulin expression and secretion can be obtained from a number of sources, such as the American Type Culture Collection ("Catalogue of Cell Lines and Hybridomas," Fifth edition (1985) Rockville, Maryland, U.S.A., which is incorporated herein by reference).

In addition to the human-like immunoglobulins specifically described herein, other "substantially homologous" modified immunoglobulins can be readily designed and manufactured utilizing various recombinant DNA techniques well known to those skilled in the art. For example, for the IL-2 receptor immunoglobulins the framework regions can vary from the sequences in Figures 3 and 4 at the primary structure level by several amino acid substitutions, terminal and intermediate additions and deletions, and the like. Moreover, a variety of different human framework regions may be used singly or in combination as a basis for the human-like immunoglobulins of the present invention. In general, modifications of the genes may be readily accomplished by a variety of well-known techniques, such as site-directed mutagenesis (see, Gillman and Smith, Gene 8:81-97 (1979) and Roberts, S. et al, Nature 328:731-734 (1987)). Alternatively, polypeptide fragments comprising only a portion of the primary antibody structure may be produced, which fragments possess one or more immunoglobulin activities (e.g., complement fixation activity). Also because like many genes, the immunoglobulin-related genes contain separate functional regions, each having one or more distinct biological activities, the genes may be fused to functional regions from other genes (e.g., enzymes) to produce fusion proteins (e.g., immunotoxins) having novel properties.

The nucleic acid sequences used in the present invention capable of ultimately expressing the desired human-like antibodies can be formed from a variety of different polynucleotides (genomic or cDNA, RNA, synthetic oligonucleotides, etc.) and components (e.g., V, J, D, and C regions), as well as by a variety of different techniques. Joining appropriate genomic sequences is presently the most common method of production, but cDNA sequences may also be utilized (see, European Patent Publication No. 0239400 and Reichmann, L., et al., Nature 332:323-327 (1988)).

As stated previously, the DNA sequences will be expressed in hosts after the sequences have been operably linked to (i.e., positioned to ensure the functioning of) an expression control sequence. These expression vectors are typically replicable in the host organisms either as episomes or as an integral part of the host chromosomal DNA. Commonly, expression vectors will contain selection markers, e.g., tetracycline or neomycin, to permit detection of those cells transformed with the desired DNA sequences (see, e.g., U.S. Patent 4,704,362).

E. coli is one prokaryotic host useful particularly for cloning the DNA sequences of the present invention. Other microbial hosts suitable for use include bacilli, such as Bacillus subtilus, and other enterobacteriaceae, such as Salmonella, Serratia, and various Pseudomonas species. In these prokaryotic hosts, one can also make expression vectors, which will typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters will typically control expression, optionally with an operator sequence, and have ribosome binding site sequences and the like, for initiating and completing transcription and translation.

Other microbes, such as yeast, may also be used for expression. Saccharomyces is a preferred host, with suitable vectors having expression control sequences, such as promoters, including 3-phosphoglycerate kinase or other glycolytic enzymes, and an origin of replication, termination sequences and the like as desired.

In addition to microorganisms, mammalian tissue cell culture may also be used to express and produce the polypeptides of the present invention (see, Winnacker, "From Genes to Clones," VCH Publishers, N.Y., N.Y. (1987), which is incorporated herein by reference). Eukaryotic cells are actually preferred, because a number of suitable host cell lines capable of secreting intact immunoglobulins have been developed in the art, and include the CHO cell lines, various COS cell lines, HeLa cells, myeloma cell lines, etc, but preferably transformed B-cells or hybridomas. Expression vectors for these cells can include expression control sequences, such as an origin of replication, a promoter, an enhancer (Queen, C., et al., Immunol. Rev. 89:49-68 (1986)), and necessary processing information sites, such as ribosome binding sites, RNA splice sites, polyadenylation sites, and transcriptional terminator sequences. Preferred expression control sequences are promoters derived from SV40 with enhancer (see, Mulligan and Berg, Science 209:1422-1427 (1980), an immunglobulin gene, Adenovirus, Bovine Papilloma Virus, and the like.

The vectors containing the DNA segments of interest (e.g., the heavy and light chain encoding sequences and expression control sequences) can be transferred into the host cell by well-known methods, which vary depending on the type of cellular host. For example, calcium chloride transfection is commonly utilized for prokaryotic cells, whereas calcium phosphate treatment or electroporation may be used for other cellular hosts. (See, generally, Maniatis, et al., Molecular Cloning: A Laboratory Manual, Cold Spring Harbor Press, (1982)).

Once expressed, the whole antibodies, their dimers, individual light and heavy chains, or other immunoglobulin forms of the present invention can be purified according to standard procedures of the art, including ammonium sulfate precipitation, affinity columns, column chromatography, gel electrophoresis and the like (see, generally, Scopes, R., Protein Purification, Springer-Verlag, N.Y. (1982)). Substantially pure immunoglobulins of at least about 90 to 95% homogeneity are preferred, and 98 to 99% or more homogeneity most preferred, for pharmaceutical uses. Once purified, partially or to homogeneity as desired, the polypeptides may then be used therapeutically (including extracorporeally) or in developing and performing assay procedures, immunofluorescent stainings, and the like. (See, generally, Immunological Methods, Vols. I and II, Lefkovits and Pernis, eds., Academic Press, New York, N.Y. (1979 and 1981)).

The IL-2 receptor specific antibodies exemplified in the present invention will typically find use individually in treating a T-cell mediated disease state. Generally, where the cell linked to a disease has been identified as IL-2 receptor bearing, then the human-like antibodies capable of blocking the binding of IL-2 to the human IL-2 receptor are suitable. For example, typical disease states suitable for treatment include graft versus host disease and transplant rejection in patient undergoing an organ transplant, such as heart, lungs, kidneys, liver, etc. Other diseases include autoimmune diseases, such as Type I diabetes, multiple sclerosis, rheumatoid arthritis, systemic lupus erythematosus, and myasthenia gravis.

The human-like antibodies made in the present invention may also be used in combination with other antibodies, particularly human monoclonal antibodies reactive with other markers on cells responsible for the disease.. For example, suitable T-cell markers can include those grouped into the so-called "Clusters of Differentiation," as named by the First International Leukocyte Differentiation Workshop, Leukocyte Typing, Bernard, et al., Eds., Springer-Verlag, N.Y. (1984).

The antibodies can also be used as separately administered compositions given in conjunction with chemotherapeutic or immunosuppressive agents. Typically, the agents will include cyclosporin A or a purine analog (e.g., methotrexate, 6-mercaptopurine, or the like), but numerous additional agents (e.g., cyclophosphamide, prednisone, etc.) well-known to those skilled in the art may also be utilized.

A preferred pharmaceutical composition comprises the use of the subject antibodies in immunotoxins. Immunotoxins are characterized by two components and are particularly useful for killing selected cells in vitro or in vivo. One component is a cytotoxic agent which is usually fatal to a cell when attached or absorbed. The second component, known as the "delivery vehicle," provides a means for delivering the toxic agent to a particular cell type, such as cells comprising a carcinoma. The two components are commonly chemically bonded together by any of a variety of well-known chemical procedures. For example, when the cytotoxic agent is a protein and the second component is an intact immunoglobulin, the linkage may be by way of heterobifunctional cross-linkers, e.g. , SPDP, carbodiimide, glutaraldehyde, or the. like. Production of various immunotoxins is well-known with the art, and can be found, for example in "Monoclonal Antibody-Toxin Conjugates: Aiming the Magic Bullet, " Thorpe et al, Monoclonal Antibodies in Clinical Medicine, Academic Press, pp. 168-190 (1982).

A variety of cytotoxic agents are suitable for use in immunotoxins. Cytotoxic agents can include radionuclides, such as Iodine-131, Yttrium-90, Rhenium-188, and Bismuth-212; a number of chemotherapeutic drugs, such as vindesine, methotrexate, adriamycin, and cisplatinm; and cytotoxic proteins such as ribosomal inhibiting proteins like pokeweed antiviral protein, Pseudomonas exotoxin A, ricin, diphtheria toxin, ricin A chain, etc., or an agent active at the cell surface, such as the phospholipase enzymes (e.g., phospholipase C). (See, generally, commonly assigned U.S.S.N. 07/290,968 filed December 28, 1988), "Chimeric Toxins," Olsnes and Phil, Pharmac. Ther., 25:355-381 (1982), and "Monoclonal Antibodies for Cancer Detection and Therapy," eds. Baldwin and Byers, pp. 159-179, 224-266, Academic Press (1985)).

The delivery component of the immunotoxin will include the human-like immunoglobulins of the present invention. Intact immunoglobulins or their binding fragments, such as Fab, are preferably used. Typically, the antibodies in the immunotoxins will be of the human IgM or IgG isotype, but other mammalian constant regions may be utilized as desired.

The human-like antibodies and pharmaceutical compositions thereof are particularly useful for parenteral administration, i.e., subcutaneously, intramuscularly or intravenously. The compositions for parenteral administration will commonly comprise a solution of the antibody or a cocktail thereof dissolved in an acceptable carrier, preferably an aqueous carrier. A variety of aqueous carriers can be used, e.g., water, buffered water, 0.4% saline, 0.3% glycine and the like. These solutions are sterile and generally free of particulate matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of antibody in these formulations can vary widely, i.e., from less than about 0.5%, usually at or at least about 1% to as much as 15 or 20% by weight and will be selected primarily based on fluid volumes, viscosities, etc., in accordance with the particular mode of administration selected.

Thus, a typical pharmaceutical composition for intramuscular injection could be made up to contain 1 ml sterile buffered water, and 50 mg of antibody. A typical composition for intravenous infusion could be made up to contain 250 ml of sterile Ringer's solution, and 150 mg of antibody. Actual methods for preparing parenterally administrable compositions will be known or apparent to those skilled in the art and are described in more detail in, for example, Remington's Pharmaceutical Science, 15th ed., Mack Publishing Company, Easton, Pennsylvania (1980).

The antibodies can be lyophilized for storage and reconstituted in a suitable carrier prior to use. This technique has been shown to be effective with conventional immune globulins and art-known lyophilization and reconstitution techniques can be employed. It will be appreciated by those skilled in the art that lyophilization and reconstitution can lead to varying degrees of antibody activity loss (e.g., with conventional immune globulins, IgM antibodies tend to have greater activity loss than IgG antibodies) and that use levels may have to be adjusted to compensate.

The compositions containing the present human-like antibodies or a cocktail thereof can be administered for prophylactic and/or therapeutic treatments. In therapeutic application, compositions are administered to a patient already suffering from a disease, in an amount sufficient to cure or at least partially arrest the disease and its complications. An amount adequate to accomplish this is defined as a "therapeutically effective dose." Amounts effective for this use will depend upon the severity of the infection and the general state of the patient's own immune system, but generally range from about 1 to about 200 mg of antibody per dose, with dosages of from 5 to 25 mg per patient being more commonly used. It must be kept in mind that the materials of this invention may generally be employed in serious disease states, that is life-threatening or potentially life-threatening situations. In such cases, in view of the minimization of extraneous substances and the lower probability of "foreign substance" rejections which are achieved by the present human-like antibodies it is possible and may be felt desirable by the treating physician to administer substantial excesses of these antibodies.

In prophylactic applications, compositions containing the present antibodies or a cocktail thereof are administered to a patient not already in a disease state to enhance the patient's resistance. Such an amount is defined to be a "prophylactically effective dose." In this use, the precise amounts again depend upon the patient's state of health and general level of immunity, but generally range from 0.1 to 25 mg per dose, especially 0.5 to 2.5 mg per patient. A preferred prophylactic use is for the prevention of kidney transplant rejection.

Single or multiple administrations of the compositions can be carried out with dose levels and pattern being selected by the treating physician. In any event, the pharmaceutical formulations should provide a quantity of the antibody(ies) of this invention sufficient to effectively treat the patient.

Human-like antibodies can further find a wide variety of utilities in vitro. By way of example, the exemplary antibodies can be utilized for T-cell typing, for isolating specific IL-2 receptor bearing cells or fragments of the receptor, for vaccine preparation, or the like.

For diagnostic purposes, the antibodies may either be labeled or unlabeled. Unlabeled antibodies can be used in combination with other labeled antibodies (second antibodies) that are reactive with the human-like antibody, such as antibodies specific for human immunoglobulin constant regions. Alternatively, the antibodies can be directly labeled. A wide variety of labels may be employed, such as radionuclides, fluors, enzymes, enzyme substrates, enzyme cofactors, enzyme inhibitors, ligands (particularly haptens), etc. Numerous types of immunoassays are available and are well known to those skilled in the art.

Kits can also be supplied for use with the subject antibodies in the protection against or detection of a cellular activity or for the presence of a selected antigen. Thus, the subject antibody composition of the present invention may be provided, usually in a lyophilized form in a container, either alone or in conjunction with additional antibodies specific for the desired cell type. The antibodies, which may be conjugated to a label or toxin, or unconjugated, are included in the kits with buffers, such as Tris, phosphate, carbonate, etc., stabilizers, biocides, inert proteins, e.g., serum albumin, or the like, and a set of instructions for use. Generally, these materials will be present in less than about 5% wt. based on the amount of active antibody, and usually present in total amount of at least about 0.001% wt. based again on the antibody concentration. Frequently, it will be desirable to include an inert extender or excipient to dilute the active ingredients, where the excipient may be present in from about 1 to 99% wt. of the total composition. Where a second antibody capable of binding to the chimeric antibody is employed in an assay, this will usually be present in a separate vial. The second antibody is typically conjugated to a label and formulated in an analogous manner with the antibody formulations described above.

The following examples are offered by way of illustration, not by limitation.

### EXPERIMENTAL

### Design of genes for human-like light and heavy chains

The sequence of the human antibody Eu (Sequences of Proteins of Immunological Interest, Kabat, E., et al., U.S. Dept. of Health and Human Services, 1983) was used to provide the framework of the humanized antibody, because the amino acid sequence of the heavy chain of anti-Tac is more homologous to the heavy chain of this antibody than to any other heavy chain sequence in the National Biomedical Foundation Protein Identification Resource.

To select the sequence of the humanized heavy chain, the anti-Tac heavy chain sequence (see, commonly assigned U.S.S.N.'s 186,862 and 223,037, which are incorporated herein by reference) was aligned with the sequence of the Eu heavy chain (Figure 1). At each position, the Eu amino acid was selected for the humanized sequence, unless that position fell in any one of the following categories, in which case the anti-Tac amino acid was selected.
(1) The position fell within a complementarity determining region (CDR), as defined by Kabat, et al., op. cit. (amino acids 31-35, 50-66, 99-106);
(2) The Eu amino acid was unusual for human heavy chains at that position, whereas the anti-Tac amino acid was typical for human heavy chains at that position (amino acids 27, 93, 95, 98, 107-109, 111);
(3) The position was immediately adjacent to a CDR in the amino acid sequence of the anti-Tac heavy chain (amino acids 30 and 67).
(4) 3-dimensional modeling of the anti-Tac antibody suggested that the amino acid was physically close to the antigen binding region (amino acids 48 and 68) . Some amino acids felL in more than one of these categories but are only listed in one.

To select the sequence of the humanized light chain, the anti-Tac light chain sequence was aligned with the sequence of the Eu light chain (Figure 2). The Eu amino acid was selected at each position, unless the position again fell into one of the categories (1) - (4), (with light chain replacing heavy chain in the category definitions):
(1) CDRs (amino acids 24-34, 50-56, 89-97).
(2) Anti-Tac amino acid more typical than Eu (amino acids 48 and 63).
(3) Adjacent to CDRs (no amino acids; Eu and anti-Tac were already the same at all these positions) .
(4) Possible 3-dimensional proximity to binding region (amino acid 60).

The actual nucleotide sequence of the heavy (Figure 3) and light chain (Figure 4) genes were selected as follows:
(1) the nucleotide sequences code for the amino acid sequences chosen as described above.
(2) 5' of these coding sequences; the nucleotide sequences code for a leader (signal) sequence, namely the leader of the light chain of the antibody MOPC 63 and the leader of the heavy chain of the antibody PCH 108A (Kabat et al., op. cit.). These leader sequences were chosen as typical of antibodies.
(3) 3' of the coding sequences, the nucleotide sequences are the sequences that follow the mouse light chain J5 segment and the mouse heavy chain J2 segment, which are part of the anti-Tac sequences. These sequences are included because they contain splice donor signals.
(4) At each end of the sequence is an Xba I site to allow cutting at the Xba I sites and cloning into the Xba I site of a vector.

### Construction of humanized light and heavy chain genes

To synthesize the heavy chain, four oligonucleotides HES12, HES13, HES14, HES15 (Figure 5A) were synthesized using an Applied Biosystems 380B DNA synthesizer. Two of the oligonucleotides are part of each strand of the heavy chain, and each oligonucleotide overlaps the next one by about 20 nucleotides to allow annealing (Figure 5B). Together, the oligonucleotides cover the entire humanized heavy chain (Figure 3) with a few extra nucleotides at each end to allow cutting at the Xba I sites. The oligonucleotides were purified from polyacrylamide gels.

Each oligonucleotide was phosphorylated using ATP and T4 polynucleotide kinase by standard procedures (see, Maniatis, op. cit.). To anneal the phosphorylated oligonucleotides, they were suspended together in 40 ul of TA (33 mM Tris acetate, pH 7.9, 66 mM potassium acetate, 10 mM magnesium acetate) at a concentration of about 3.75 uM each, heated to 95 deg for 4 min. and cooled slowly to 4 deg. To synthesize the complete gene from the oligonucleotides by synthesizing the opposite strand of each oligonucleotide (Figure 5B), the following components were added in a final volume of 100ul:

| | |
|---|---|
| 10 ul | annealed oligonucleotides |
| 0.16 mM each | deoxyribonucleotide |
| 0.5 mM | ATP |
| 0.5 mM | DTT |
| 100 ug/ml | BSA |
| 3.5 ug/ml | T4 g43 protein (DNA polymerase) |
| 25 ug/ml | T4 g44/62 protein (polymerase accessory protein) |
| 25 ug/ml | 45 protein (polymerase accessory protein) |

The mixture was incubated at 37 deg for 30 min. Then 10 u of T4 DNA ligase was added and incubation at 37 deg resumed for 30 min. The polymerase and ligase were inactivated by incubation of the reaction at 70 deg for 15 min. To digest the gene with Xba I, to the reaction was added 50 ul of 2x TA containing BSA at 200 ug/ml and DTT at 1 mM, 43 ul of water, and 50 u of Xba I in 5 ul. The reaction was incubated for 3 hr at 37 deg, and run on a gel. The 431 bp Xba I fragment was purified from a gel and cloned into the Xba I site of the plasmid pUC19 by standard methods. Four plasmid isolates were purified and sequenced using the dideoxy method. One of these had the correct sequence (Figure 3).

To synthesize the light chain, four oligonucleotides JFD1, JFD2, JFD3, JFD4 (Figure 6A) were synthesized. Two of the oligonucleotides are part of each strand of the light chain, and each oligonucleotide overlaps the next one by about 20 nucleotides to allow annealing (Figure 6B). Together, the oligonucleotides cover the entire humanized light chain (Figure 4) with a few extra nucleotides at each end to allow cutting at the Xba I sites. The oligonucleotides were purified from polyacrylamide gels.

The light chain gene was synthesized from these olignucleotides in two parts. 0.5 ug each of JFD1 and JFD2 were combined in 20 ul sequenase buffer (40 mM Tris-HCl, pH 7.5, 20 mM magnesium chloride, 50 mM sodium chloride), heated at 70 deg for 3 min and allowed to cool slowly to 23 deg in order for the oligonucleotides to anneal. JFD3 and JFD4 were treated in the same way. Each reaction was made 10 mM in DTT and 0.5 mM in each deoxyribonucleotide and 6.5 u of sequenase (US Biochemicals) was added, in a final volume of 24 ul, and incubated for 1 hr at 37 deg to synthesize the opposite strands of the oligonucleotides. Xba I and Hind III were added to each reaction to digest the DNA (there is a Hind III site in the region where JFD2 and JFD3 overlap and therefore in each of the synthesized DNAs; Figure 6B). The reactions were run on polyacrylamide gels, and the Xba I - Hind III fragments were purified and cloned into pUC18 by standard methods. Several plasmid isolates for each fragment were sequenced by the dideoxy method, and correct ones chosen.

### Construction of plasmids to express humanized light and heavy chains

The heavy chain Xba I fragment was isolated from the pUC19 plasmid in which it had been inserted and then inserted into the Xba I site of the vector pVγ1 (see, commonly assigned U.S.S.N. 223,037) in the correct orientation by standard methods, to produce the plasmid pHuGTAC1 (Figure 7). This plasmid will express high levels of a complete heavy chain when transfected into an appropriate host cell.

The two light chain Xba I - Hind III fragments were isolated from the pUC18 plasmids in which they had been inserted. The vector plasmid pVκ1 (see, commonly assigned U.S.S.N. 223,037) was cut with Xba I, dephosphorylated and ligated with the two fragments by standard methods. The desired reaction product has the circular form: vector - Xba I - fragment 1 - Hind III - fragment 2 - Xba I - vector. Several plasmid isolates were analyzed by restriction mapping and sequencing, and one with this form chosen. This plasmid, pHuLTAC (Figure 8), therefore contains the complete humanized light chain (Figure 4) and will express high levels of the light chain when transfected into an appropriate host cell.

### Synthesis and affinity of humanized antibody

The plasmids pHuGTAC1 and pHuLTAC were transfected into mouse Sp2/0 cells, and cells that integrated the plasmids were selected on the basis of resistance to mycophenolic acid and/or hygromycin B conferred by the gpt and hyg genes on the plasmids (Figures 7, 8) by standard methods. To verify that these cells secreted antibody that binds to the IL-2 receptor, supernatant from the cells was incubated with HUT-102 cells that are known to express the IL-2 receptor. After washing, the cells were incubated with fluorescein-conjugated goat anti-human antibody, washed, and analyzed for fluorescence on a FACSCAN cytofluorometer. The results (Figure 9A), clearly show that the humanized antibody binds to these cells, but not to Jurkat T-cells that do not express the IL-2 receptor (Figure 9D). As controls, the original mouse anti-Tac antibody was also used to stain these cells (Figure 9B,C), giving similar results.

For further experiments, cells producing the humanized antibody were injected into mice, and the resultant ascites collected. Humanized antibody was purified to substantial homogeneity from the ascites by passage through an affinity column of goat anti-human immunoglobulin antibody, prepared on an Affigel-10 support (Bio-Rad Laboratories, Inc., Richmond, CA) according to standard techniques. To determine the affinity of the humanized antibody relative to the original anti-Tac antibody, a competitive binding experiment was performed. About 5 x 10⁵ HUT-102 cells were incubated with known quantities (10 - 40 ng) of the anti-Tac antibody and the humanized anti-Tac antibody for 10 min at 4 deg. Then 100 ng of biotinylated anti-Tac was added to the cells and incubated for 30 min at 4 deg. This quantity of anti-Tac had previously been determined to be sufficient to saturate the binding sites on the cells, but not to be in large excess. Then the cells were washed twice with 2 ml of phosphate buffered saline (PBS) containing 0.1% sodium azide. The cells were then incubated for 30 min at 4 deg with 250 ng of phycoerythrin-conjugated avidin, which bound to the biotinylated anti-Tac already bound to the cells. The cells were washed again as above, fixed in PBS containing 1% paraformaldehyde, and analyzed for fluorescence on a FACSCAN cytofluorometer.

Use of increasing amounts (10 - 40 ng) of the anti-Tac antibody as competitor in the first step decreased the amount of biotinylated anti-Tac that could bind to the cells in the second step, and therefore the amount of phycoerythrin-conjugated avidin that bound in the last step, thus decreasing fluorescence (Figure 10A). Equivalent amounts (20 ng) of anti-Tac, and humanized anti-Tac used as competitor decreased the fluorescence to approximately the same degree (Figure 10B). This shows that these antibodies have approximately the same affinity (within 3 to 4 fold), because if one had much greater affinity, it would have more effectively competed with the biotinylated anti-Tac, thus decreasing fluorescence more.

### Biological properties of the humanized antibody

For optimal use in treatment of human disease, the humanized antibody should be able to destroy T-cells in the body that express the IL-2 receptor. One mechanism by which antibodies may destroy target cells is antibody-dependent cell-mediated cytotoxicity, abbreviated ADCC (Fundamental Immunology, Paul, W., Ed., Raven Press, New York (1984), at pg. 681), in which the antibody forms a bridge between the target cell and an effector cell such as a macrophage that can lyse the target. To determine whether the humanized antibody and the original mouse anti-Tac antibody can mediate ADCC, a chromium release assay was performed by standard methods. Specifically, human leukemia HUT-102 cells, which express the IL-2 receptor, were incubated with ⁵¹Cr to allow them to absorb this radionuclide. The HUT-102 cells were then incubated with an excess of either anti-Tac or humanized anti-Tac antibody. The HUT-102 cells were next incubated for 4 hrs with either a 30:1 or 100:1 ratio of effector cells, which were normal purified human peripheral blood mononuclear cells that had been activated by incubation for about 20 hrs with human recombinant IL-2. Release of ⁵¹Cr, which indicated lysis of the target HUT-102 cells, was measured and the background subtracted (Table 1). The results show that at either ratio of effector cells, anti-Tac did not lyse a significant number of the target cells (less than 5%), while the humanized antibody did (more than 20%). Hence, the humanized antibody is likely to be more efficacious than the original mouse antibody in treating T-cell leukemia or other T-cell mediated diseases.

**TABLE 1**

| Percent ⁵¹Cr release after ADCC | | |
|---|---|---|
| | Effector: Target ratio | |
| | 30:1 | 100:1 |
| Antibody | | |
| Anti-Tac | 4% | < 1% |
| Humanized anti-Tac | 24% | 23% |

From the foregoing, it will be appreciated that the human-like immunoglobulins of the present invention offer numerous advantages of other antibodies. For example, in comparison to anti-Tac mouse monoclonal antibodies, the present human-like IL-2 receptor immunoglobulins can be more economically produced and contain substantially less foreign amino acid sequences. This reduced likelihood of antigenicity after injection into a human patient represents a significant therapeutic improvement for immunoglobulins designed in accordance with the above criteria.

Although the present invention has been described in some detail by way of illustration and example for purposes of clarity and understanding, it will be apparent that certain changes and modifications may be practiced within the scope of the appended claims.

## Claims

1. A process for producing a humanized immunoglobulin suitable for an immunotoxin in which immunotoxin said immunoglobulin is chemically bonded to a cytotoxic agent, wherein said humanized immunoglobulin binds to an antigen and has a human acceptor framework region and Kabat complementarity determining regions (CDRs) from a donor immunoglobulin, further wherein the production of said humanized immunoglobulin comprises substituting at least one non-CDR amino acid from the donor immunoglobulin light chain framework for the corresponding amino acid in the acceptor framework, at a position where:
(a) the amino acid in the acceptor framework is rare for said position and the corresponding amino acid in the donor framework is common for said position in human immunoglobulin sequences; or
(b) the amino acid is immediately adjacent to one of the CDRs; or
(c) the amino acid is predicted to have a side chain atom capable of interacting with the CDRs of the humanized immunoglobulin.

2. The process of claim 1, wherein the humanized immunoglobulin has a binding affinity for the antigen of 10⁸ M⁻¹ or higher.

3. The process of claim 1 or claim 2 wherein the humanized immunoglobulin is an antibody tetramer composed of an identical pair of heavy chain polypeptides and an identical pair of light chain polypeptides.

4. The process of claims 1 to 3, wherein the cytotoxic agent is a protein, radionuclide or chemotherapeutic drug.

5. The process of claim 4, wherein the cytotoxic agent is a protein and wherein said protein is chemically bonded to the humanized immunoglobulin by way of a heterobifunctional cross-linker, e.g. SPDP, carbodimide or glutaraldehyde.

6. The process of claim 4, wherein the cytotoxic agent is a radionuclide such as lodine-131, Yttrium-90, Rehnium-188 or Bismuth-212.

7. The process of claim 4, wherein the cytotoxic agent is a chemotherapeutic drug such as vindesine, methotrexate, adriamycin or cisplatinum.

8. The process of claims 1 to 7, wherein said donor immunoglobulin is a mouse immunoglobulin.

9. The process of claims 1 to 8, wherein said humanized immunoglobulin has a light chain constant region encoded by a kappa or lambda constant region gene.

10. The process of claims 1 to 9, wherein said humanized immunoglobulin has a heavy chain constant region encoded by an alpha, gamma, delta, epsilon or mu constant region gene.

## Patentansprüche

1. Verfahren zur Herstellung eines humanisierten Immunglobulins, das als Immuntoxin geeignet ist, wobei das Immunglobulin in dem Immuntoxin chemisch an einen zytotoxischen Wirkstoff gebunden ist, wobei das humanisierte Immunglobulin an ein Antigen bindet und eine humane Akzeptor-Rahmenregion und Kabatkomplementaritätsbestimmende Regionen (CDRs) von einem Donor-Immunglobulin aufweist, und wobei die Herstellung des humanisierten Immunglobulins die Substitution von mindestens einer Nicht-CDR-Aminosäure von der Rahmenregion der leichten Kette des Donor-Immunglobulins anstelle der entsprechenden Aminosäure in der Akzeptor-Rahmenregion an Positionen umfasst, an denen:
(a) die Aminosäure in der Akzeptor-Rahmenregion für diese Position selten ist und die korrespondierende Aminosäure in der Donor-Rahmenregion für diese Position in humanen Immunglobulin-Sequenzen häufig ist; oder
(b) die Aminosäure unmittelbar benachbart zu einer der CDRs ist; oder
(c) für die Aminosäure ein Seitenkettenatom vorhergesagt wird, das fähig ist, mit den CDRs des humanisierten Immunglobulins zu interagieren.

2. Verfahren nach Anspruch 1, wobei das humanisierte Immunglobulin für das Antigen eine Bindungsaffinität von 10⁸ m⁻¹ oder höher hat.

3. Verfahren nach Anspruch 1 oder 2, wobei das humanisierte Immunglobulin ein Antikörper-Tetramer ist, das aus einem identischen Paar von schwere Ketten Polypeptiden und einem identischen Paar von leichte Ketten Polypeptiden aufgebaut ist.

4. Verfahren nach einem der Ansprüche 1 bis 3, wobei der zytotoxische Wirkstoff ein Protein, ein Radionuklid oder ein chemotherapeutischer Wirkstoff ist.

5. Verfahren nach Anspruch 4, wobei der zytotoxische Wirkstoff ein Protein ist und wobei das Protein über einen hetero-bifunktionalen Vernetzer, wie etwa SPDP, Carbodiimid oder Glutaraldehyd chemisch mit dem humanisierten Immunglobulin verbunden ist.

6. Verfahren nach Anspruch 4, wobei der zytotoxische Wirkstoff ein Radionuklid, wie etwa Jod-131, Yttrium-90, Rhenium-188 oder Bismut-212 ist.

7. Verfahren nach Anspruch 4, wobei der zytotoxische Wirkstoff ein chemotherapeutischer Wirkstoff, wie etwa Vindesin, Methotrexat, Adriamycin oder Cisplatinum ist.

8. Verfahren nach einem der Ansprüche 1 bis 7, wobei das Donor-Immunglobulin ein Maus-Immunglobulin ist.

9. Verfahren nach einem der Ansprüche 1 bis 8, wobei das humanisierte Immunglobulin eine konstante Region der leichten Kette aufweist, die von einem Gen für eine Kappa- oder Lambda-konstante Region kodiert wird.

10. Verfahren nach einem der Ansprüche 1 bis 9, wobei das humanisierte Immunglobulin eine konstante Region der schweren Kette aufweist, die von einem Gen für eine Alpha-, Gamma-, Delta-, Epsilon- oder Mu-konstante Region kodiert wird.

## Revendications

1. Un processus de production d'une immunoglobuline humanisée adapté à une immunotoxine dans laquelle immunotoxine ladite immunoglobuline est chimiquement liée à un agent cytotoxique, où ladite immunoglobuline humanisée se lie à un antigène et possède une région charpente d'accepteur humain et des régions déterminantes de complémentarité (CDR) Kabat provenant d'une immunoglobuline donneuse, où en outre la production de ladite immunoglobuline humanisée comprend la substitution d'au moins un acide aminé non CDR provenant de la charpente à chaîne légère de l'immunoglobuline donneuse pour l'acide aminé correspondant dans la charpente d'accepteur, au niveau d'une position dans laquelle :
(a) l'acide aminé dans la charpente d'accepteur est rare pour ladite position et l'acide aminé correspondant dans la charpente donneuse est commun pour ladite position dans des séquences d'immunoglobuline humaine, ou
(b) l'acide aminé est immédiatement adjacent à l'une des CDR, ou
(c) l'acide aminé est prévu avoir un atome de chaîne latérale capable d'interagir avec les CDR de l'immunoglobuline humanisée.

2. Le processus selon la Revendication 1, où l'immunoglobuline humanisée possède une affinité de liaison pour l'antigène de 10⁸ M⁻¹ ou plus.

3. Le processus selon la Revendication 1 ou 2 où l'immunoglobuline humanisée est un tétramère d'anticorps composé d'une paire identique de polypeptides à chaîne lourde et d'une paire identique de polypeptides à chaîne légère.

4. Le processus selon l'une quelconque des Revendications 1 à 3, où l'agent cytotoxique est une protéine, un radionucléide ou un médicament chimiothérapeutique.

5. Le processus selon la Revendication 4, où l'agent cytotoxique est une protéine et où ladite protéine est chimiquement liée à l'immunoglobuline humanisée au moyen d'un agent de réticulation hétérobifonctionnel, par exemple SPDP, carbodimide ou glutaraldéhyde.

6. Le processus selon la Revendication 4, où l'agent cytotoxique est un radionucléide tel que Iode-131, Yttrium-90, Rhénium-188 ou Bismuth-212.

7. Le processus selon la Revendication 4, où l'agent cytotoxique est un médicament chimiothérapeutique tel que vindésine, méthotrexate, adriamycine ou cisplatine.

8. Le processus selon l'une quelconque des Revendications 1 à 7, où ladite immunoglobuline donneuse est une immunoglobuline de souris.

9. Le processus selon l'une quelconque des Revendications 1 à 8, où ladite immunoglobuline humanisée possède une région constante à chaîne légère codée par un gène de région constante kappa ou lambda.

10. Le processus selon l'une quelconque des Revendications 1 à 9, où ladite immunoglobuline humanisée possède une région constante à chaîne lourde codée par un gène de région constante alpha, gamma, delta, epsilon ou mu.
